(19) Europäisches Patentamt
European Patent Office
Office européen des brevets

(11) **EP 4 008 309 A1**

(12) **EUROPEAN PATENT APPLICATION**
published in accordance with Art. 153(4) EPC

(43) Date of publication:
**08.06.2022 Bulletin 2022/23**

(21) Application number: **20850549.5**

(22) Date of filing: **31.07.2020**

(51) International Patent Classification (IPC):
**A61K 9/06** (2006.01)  **A61K 47/38** (2006.01)
**A61K 47/36** (2006.01)  **A61K 47/42** (2017.01)
**A61K 47/32** (2006.01)  **A61K 31/475** (2006.01)
**A61P 19/02** (2006.01)  **A61P 29/00** (2006.01)

(52) Cooperative Patent Classification (CPC):
**A61K 9/06; A61K 31/475; A61K 47/32;**
**A61K 47/36; A61K 47/38; A61K 47/42;**
**A61P 19/02; A61P 29/00**

(86) International application number:
**PCT/CN2020/106108**

(87) International publication number:
**WO 2021/023099 (11.02.2021 Gazette 2021/06)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **02.08.2019 CN 201910709653**

(71) Applicant: **Beijing Increasepharm Corporation Limited**
**Beijing 102200 (CN)**

(72) Inventors:
• **ZHANG, Baoxian**
**Beijing 102200 (CN)**

• **HU, Jie**
**Beijing 102200 (CN)**
• **WU, Zhenzhen**
**Beijing 102200 (CN)**
• **ZHAO, Qian**
**Beijing 102200 (CN)**
• **XUE, Chunmei**
**Beijing 102200 (CN)**
• **LI, Wenhui**
**Beijing 102200 (CN)**
• **CAO, Ying**
**Beijing 102200 (CN)**

(74) Representative: **ABG Intellectual Property Law, S.L.**
**Avenida de Burgos, 16D**
**Edificio Euromor**
**28036 Madrid (ES)**

(54) **BRUCINE GEL FORMULATION AND PREPARATION METHOD THEREFOR**

(57) A brucine gel formulation, a preparation method therefor and use thereof. Said gel formulation comprises 0.5%-1% of brucine, 0.5%-3% of a gel matrix and 10%-30% of a co-solvent. Said brucine hydrogel does not contain a transdermal enhancer, but has an excellent transdermal effect, is easy to coat, has good biosolubility, good skin absorption and good drug film adhesion, and is non-irritating to the skin and mucosa.

EP 4 008 309 A1

**Description**

**CROSS REFERENCE**

This application claims the priority of Chinese Patent Application No. 201910709653.x, filed with the China National Intellectual Property Administration on August 2, 2019, and titled with "BRUCINE GEL FORMULATION AND PREPARATION METHOD THEREFOR", which is hereby incorporated by reference.

**FIELD**

The present disclosure relates to the field of pharmaceutical formulations, and specifically relates to a brucine gel formulation and a preparation method and use thereof.

**BACKGROUND**

Knee osteoarthritis is a disease based on degenerative pathological changes. When knee osteoarthritis occurs, the pain is severe and shows the characteristics of local severity and deep location. It requires drugs to quickly reach the diseased site and have a lasting effect.

*Strychnos nux-vomica* L. is bitter and warm in nature, and is very poisonous. It has the effects of activating collaterals, relieving pain, resolving masses and reducing swelling, and is used for traumatic injury, swelling and pain from fractures, rheumatism and arthralgia, numbness, paralysis, etc. The main active ingredients of *Strychnos nux-vomica* L. are total alkaloids, including brucine, strychnine, vomicine, novacine, loganin, icajine, etc.; among them, brucine has anti-inflammatory and analgesic effects; while strychnine basically has no anti-inflammatory and analgesic effects, and strychnine is the main toxic ingredient of *Strychnos nux-vomica* L..

The pharmacokinetic studies of *Strychnos nux-vomica* L. and its formulations show that brucine can be quickly eliminated after absorption with a short half-life, which is not conducive to the analgesic effect. Moreover, the therapeutic dose of *Strychnos nux-vomica* L. is close to the toxic dose, which is difficult to control the optimal dose by oral administration, greatly limiting its clinical application.

At present, the related formulations of *Strychnos nux-vomica* L. that have been put on the market are oral Shisanwei Maqianzi Wan and Maqianzi San. The active ingredients in the related formulations are either the extracts of *Strychnos nux-vomica* L., which are either the total alkaloids of *Strychnos nux-vomica* L., or comprise both brucine and strychnine. These formulations or dosages are difficult to accurately control, resulting in the therapeutic dose and the toxic dose being close to each other, or they contain strychnine, a toxic ingredient. There are few related externally applied formulations that only contain brucine monomer as the active ingredient. The research trend of brucine is how to develop it into a safe and effective new medicinal dosage form to give full play to its analgesic, anti-inflammatory, anti-cancer and other biological activities, while avoiding central toxicity and better serve the clinic.

For gel formulations, how to improve the transdermal effect of active ingredients is a major problem. Currently, penetration enhancers, such as azone, are usually added to the formula of gel formulations to improve the transdermal effect of the active ingredients. Azone has a strong skin penetration promoting effect, but due to the high lipophilicity, it will accumulate in the skin due to long-term use, which will destroy the stratum corneum, cause irreversible skin damage, and have a strong irritation. The inventors found that for the gel formulation of single brucine, the choice of excipients, especially the gel matrix material (gel skeleton material), can greatly affect the pharmacokinetics of the resulting formulation. Many commonly used matrix materials are not suitable for the preparation of brucine gel formulations, and the transdermal effect is often not ideal, or there are other shortcomings in the properties of the preparation, such as stability and viscosity.

Therefore, there is an urgent need in the prior art for a transdermal preparation of brucine with safety and excellent transdermal effect.

**SUMMARY**

The purpose of the present disclosure is to provide a new brucine gel formulation with safety and excellent transdermal effect and preparation method and use thereof in view of the defects of the prior art.

In the first aspect of the present disclosure, there is provided a brucine gel formulation, wherein by weight percentage, the gel formulation comprises 0.5%-1% of brucine, 0.5%-3% of gel matrix material and 10%-30% of a co-solvent.

In the aforementioned brucine gel formulation, the gel matrix material is one of carbomer, sodium carboxymethyl cellulose, hydroxypropyl methyl cellulose (HPMC), sodium alginate, gum arabic, gum tragacanth, gelatin, chitosan, and polyacrylic acid and sodium salt thereof, or a combination thereof; preferably, the gel matrix material is selected from the group consisting of carbomer, sodium carboxymethyl cellulose, hydroxypropyl methyl cellulose, gum arabic and sodium polyacrylate; more preferably, the gel matrix material is selected from carbomer, sodium polyacrylate and hydroxypropyl methylcellulose; more preferably, the gel matrix material is selected from carbomer and hydroxypropyl methylcellulose; and particularly preferably, the gel matrix material is hydroxypropyl methyl cellulose.

In the aforementioned brucine gel formulation, the co-solvent is one of ethanol, benzyl alcohol, propylene glycol, glycerin, polyethylene glycol 400 and isopropanol, or a combination thereof.

In the aforementioned brucine gel formulation, the gel formulation can further comprise a pH adjusting agent, preferably, the pH adjusting agent is selected from the group consisting of an organic amine, sodium hydroxide, sodium bicarbonate, sodium carbonate, acetic acid, citric acid and phosphate buffer; more preferably, the organic amine is selected from triethanolamine, triethylamine, diethylamine and laurylamine.

In the aforementioned brucine gel formulation, by weight percentage, the gel formulation can further comprise 0.01%-0.1% of an antioxidant, and the antioxidant is preferably one of anhydrous sodium sulfite, sodium metabisulfite, sodium bisulfite, sodium thiosulfate, tert-butyl p-hydroxyanisole, dibutyl phenol, vitamin C, vitamin C palmitate, and ethylenediaminetetraacetic acid and sodium salt thereof, or a combination thereof.

In the aforementioned brucine gel formulation, by weight percentage, the gel formulation can further comprise 0.01%-0.15% of a bacteriostatic agent, and the bacteriostatic agent is preferably one of chlorobutanol, ethyl paraben, benzyl alcohol, methyl paraben, butyl paraben, sorbic acid and potassium salt thereof, and benzoic acid and sodium salt thereof, or a combination thereof.

In the aforementioned brucine gel formulation, by weight percentage, the gel formulation can further comprise 68%-80% of water.

In another aspect of the present disclosure, there is provided a method for preparing the gel formulation of the present disclosure, comprising steps of: (1) swelling the gel matrix material to obtain a gel matrix I; (2) dissolving brucine and a co-solvent in water to obtain a brucine solution II; (3) mixing the gel matrix I and the brucine solution II to obtain the brucine gel formulation.

Wherein, according to the different pH of the gel matrix, step (1) can further comprise adding a pH adjusting agent to adjust pH value to 5.5-6.5, practically by adding a pH adjusting agent to a solvent used for swelling.

Step (2) can further comprise adding an antioxidant and/or a bacteriostatic agent and/or a transdermal agent, practically by adding and dissolving the antioxidant, bacteriostatic agent and/or transdermal agent in water.

According to the determination of pH value of the brucine gel formulation, step (3) can further comprise adding a pH adjusting agent to adjust pH value to 6.5-7.5.

The aforementioned preparation method only enumerates the preparation method of the present disclosure, and it should not be understood that the preparation method of the present disclosure is only limited to the above-listed method.

In the third aspect of the present disclosure, there is provided use of the brucine gel formulation in the manufacture of a medicament for treating knee osteoarthritis.

Compared with the prior art, the present disclosure has the following beneficial effects:

1. Oral administration of brucine for the treatment of arthritis has great toxic and side effects. The development of a gel formulation of brucine for topical application can avoid oral toxicity, improve the bioavailability of the drug, and improve the therapeutic effect.

2. The brucine gel formulation of the present disclosure employs simple pharmaceutical excipients, has excellent transdermal effect without penetration enhancers, easy coating, good biocompatibility and skin absorption, good drug film adhesion, and no irritation to skin and mucosa.

DETAILED DESCRIPTION

The embodiments of the present disclosure will be described in detail below in conjunction with examples. However, those skilled in the art will understand that the following examples are only used to illustrate the present disclosure and should not be regarded as limiting the scope of the present disclosure. If specific conditions are not indicated in the examples, it can be carried out in accordance with the conventional conditions or the conditions recommended by the manufacturer. The reagents or instruments used without indicating the manufacturer are all conventional products that can be purchased commercially.

Example 1

| Formula | Dosage (g) |
|---|---|
| Brucine | 10.0 |
| Carbomer | 8.3 |
| absolute ethanol | 166.7 |
| Propylene glycol | 250.0 |
| 5M sodium hydroxide | 0.05 |
| Ethylenediaminetetraacetic acid | 0.83 |
| Ethyl paraben | 1.67 |
| Water | 1230.0 |

Preparation method:

(1) 8.3 g of carbomer was added into water to swell completely, and pH was adjusted to about 6 with 5M sodium hydroxide to obtain gel matrix I; (2) 1.67 g of ethyl paraben was dissolved in propylene glycol to obtain solution a, 0.83 g of ethylenediaminetetraacetic acid was dissolved in an appropriate amount of water to obtain solution b, 10 g of brucine was dissolved in absolute ethanol and the solution a to obtain solution c, and the solution c was mixed with the solution b evenly to obtain brucine solution II; (3) the gel matrix I was mixed with the brucine solution II and stirred evenly to obtain a brucine gel formulation.

Example 2

| Formula | Dosage (g) |
|---|---|
| Brucine | 10.0 |
| Hydroxypropyl methylcellulose | 33.33 |
| absolute ethanol | 250.0 |
| Propylene glycol | 250.0 |
| 0.1M citric acid | 0.08 |
| Ethylenediaminetetraacetic acid | 0.83 |
| Ethyl paraben | 1.67 |
| Water | 1120.0 |

Preparation method:

(1) 33.33 g of hydroxypropyl methylcellulose was added into water to swell completely to obtain gel matrix I; (2) 1.67 g of ethyl paraben was dissolved in propylene glycol to obtain solution a, 0.83 g of ethylenediaminetetraacetic acid was dissolved in an appropriate amount of water to obtain solution b, 10.0 g of brucine was dissolved in absolute ethanol and the solution a to obtain solution c, and the solution c was mixed with the solution b evenly to obtain brucine solution II; (3) the gel matrix I was mixed with the brucine solution II and stirred evenly, and 0.1M citric acid was added to adjust pH to about 7 to obtain a brucine gel formulation.

Example 3

| Formula | Dosage (g) |
|---|---|
| Brucine | 10.0 |

(continued)

| Formula | Dosage (g) |
|---|---|
| Hydroxypropyl methylcellulose | 40.0 |
| absolute ethanol | 200.0 |
| Propylene glycol | 300.0 |
| 0.1M citric acid | 0.10 |
| Water | 1450.0 |

Preparation method:

(1) 40 g of hydroxypropyl methylcellulose was added into water to swell completely to obtain gel matrix I; (2) 10.0 g of brucine was dissolved in absolute ethanol and propylene glycol, mixed with water at a balanced amount in the formula and stirred evenly to obtain brucine solution II; (3) the gel matrix I was mixed with the brucine solution II and stirred evenly, and 0.1M citric acid was added to adjust pH to about 7 to obtain a brucine gel formulation.

Example 4

| Formula | Dosage (g) |
|---|---|
| Brucine | 10.0 |
| Carbomer | 10.0 |
| absolute ethanol | 200.0 |
| Propylene glycol | 300.0 |
| 5M sodium hydroxide | 0.04 |
| Water | 1480.0 |

Preparation method:

(1) 10 g of carbomer was added into water to swell completely, and pH was adjusted to about 6 with 5M sodium hydroxide to obtain gel matrix I; (2) 10 g of brucine was dissolved in absolute ethanol and propylene glycol, mixed with water at a balanced amount in the formula and stirred evenly to obtain brucine solution II; (3) the gel matrix I was mixed with the brucine solution II and stirred evenly to obtain a brucine gel formulation.

Example 5

| Formula | Dosage (g) |
|---|---|
| Brucine | 10.0 |
| Sodium polyacrylate | 20.0 |
| Absolute ethanol | 200.0 |
| Propylene glycol | 300.0 |
| Water | 1470.0 |

Preparation method:

(1) 20 g of polyacrylic acid was added into water to swell completely to obtain gel matrix I; (2) 10 g of brucine was dissolved in absolute ethanol and propylene glycol, mixed with water at a balanced amount in the formula and stirred evenly to obtain brucine solution II; (3) the gel matrix I was mixed with the brucine solution II and stirred evenly to obtain a brucine gel formulation.

Example 6

| Formula | Dosage (g) |
|---|---|
| Brucine | 10.0 |
| Sodium carboxymethyl cellulose | 30.0 |
| Absolute ethanol | 100.0 |
| Glycerin | 100.0 |
| Water | 760.0 |

Preparation method:

(1) 30 g of sodium carboxymethyl cellulose was added into water to swell completely to obtain gel matrix I; (2) 10 g of brucine was dissolved in absolute ethanol and glycerin, mixed with water at an amount remaining in the formula and stirred evenly to obtain brucine solution II; (3) the gel matrix I was mixed with the brucine solution II and stirred evenly to obtain a brucine gel formulation.

Example 7

| Formula | Dosage (g) |
|---|---|
| Brucine | 10.0 |
| Hydroxypropyl methylcellulose | 25.0 |
| Polyethylene glycol 400 | 300.0 |
| 0.1M citric acid | 0.02 |
| Water | 665.0 |

Preparation method:

(1) 25 g of hydroxypropyl methyl cellulose was added into water to swell completely to obtain gel matrix I; (2) 10 g of brucine was dissolved in polyethylene glycol 400, mixed with water at a balanced amount in the formula and stirred evenly to obtain brucine solution II; (3) the gel matrix I was mixed with the brucine solution II, and 0.1M citric acid was added to adjust pH to about 7 and stirred evenly to obtain a brucine gel formulation.

Example 8

| Formula | Dosage (g) |
|---|---|
| Brucine | 10.0 |
| Gum arabic | 8.33 |
| Benzyl alcohol | 167.0 |
| Water | 1480.0 |

Preparation method:

(1) 8.33 g of gum arabic was added into water to swell completely to obtain gel matrix I; (2) 10 g of brucine was dissolved in benzyl alcohol, mixed with water at a balanced amount in the formula and stirred evenly to obtain brucine solution II; (3) the gel matrix I was mixed with the brucine solution II and stirred evenly to obtain a brucine gel formulation.

Example 9

| Formula | Dosage (g) |
|---|---|
| Brucine | 10.0 |
| Carbomer | 30.0 |
| Glycerin | 100.0 |
| Isopropanol | 400.0 |
| 5M sodium hydroxide | 0.06 |
| Water | 1460.0 |

Preparation method:

(1) 30 g of carbomer was added into water to swell completely, and pH was adjusted to about 6 with 5M sodium hydroxide to obtain gel matrix I; (2) 10 g of brucine was dissolved in glycerin and isopropanol, mixed with water at a balanced amount in the formula and stirred evenly to obtain brucine solution II; (3) the gel matrix I was mixed with the brucine solution II and stirred evenly to obtain a brucine gel formulation.

Example 10

| Formula | Dosage (g) |
|---|---|
| Brucine | 10.0 |
| Hydroxypropyl methylcellulose | 30.0 |
| Glycerin | 100.0 |
| 0.1M citric acid | 0.02 |
| Water | 880.0 |

Preparation method:

(1) 30 g of hydroxypropyl methyl cellulose was added into water to swell completely to obtain gel matrix I; (2) 10 g of brucine was dissolved in glycerin, mixed with water at a balanced amount in the formula and stirred evenly to obtain brucine solution II; (3) the gel matrix I was mixed with the brucine solution II, and 0.1M citric acid was added to adjust pH to about 7 and stirred evenly to obtain a brucine gel formulation.

Comparative Example 1

| Formula | Dosage (g) |
|---|---|
| Brucine | 10.0 |

(continued)

| Formula | Dosage (g) |
|---|---|
| Polyvinyl alcohol | 40.0 |
| absolute ethanol | 200.0 |
| Propylene glycol | 300.0 |
| 0.1M citric acid | 0.04 |
| Sodium hyaluronate | 20.0 |
| Water | 1430.0 |

Preparation method:

(1) 40.0 g of polyvinyl alcohol and 20 g of sodium hyaluronate were added into water to swell completely to obtain gel matrix I; (2) 10 g of brucine was dissolved in absolute ethanol and propylene glycol, mixed with water at a balanced amount in the formula and stirred evenly to obtain brucine solution II; (3) the gel matrix I was mixed with the brucine solution II and stirred evenly, and 0.1M citric acid was added to adjust pH to about 7 and stirred evenly to obtain a brucine gel formulation.

Comparative Example 2

| Formula | Dosage (g) |
|---|---|
| total alkaloids of *Strychnos nux-vomica* L. | Containing 10.0 g brucine and 10.0g strychnine |
| Hydroxypropyl methylcellulose | 40.0 |
| absolute ethanol | 200.0 |
| Propylene glycol | 300.0 |
| 0.1M citric acid | 0.10 |
| Water | 1440.0 |

Preparation method:

(1) 40.0 g of hydroxypropyl methyl cellulose was added into water to swell completely to obtain gel matrix I; (2) 20 g of total alkaloids of *Strychnos nux-vomica* L. was dissolved in absolute ethanol and propylene glycol, mixed with water at a balanced amount in the formula and stirred evenly to obtain brucine solution II; (3) the gel matrix I was mixed with the brucine solution II and stirred evenly, and 0.1M citric acid was added to adjust pH to about 7 to obtain a brucine gel formulation.

Comparative Example 3

| Formula | Dosage (g) |
|---|---|
| Brucine | 10.0 |
| Gum tragacanth | 40.0 |
| Absolute ethanol | 200.0 |
| Propylene glycol | 300.0 |
| 0.1M citric acid | 0.06 |

(continued)

| Formula | Dosage (g) |
|---------|------------|
| Water | 1450.0 |

Preparation method:

(1) 40 g of gum tragacanth was added into water to swell completely to obtain gel matrix I; (2) 10 g of brucine was dissolved in absolute ethanol and propylene glycol, mixed with water at a balanced amount in the formula and stirred evenly to obtain brucine solution II; (3) the gel matrix I was mixed with the brucine solution II and stirred evenly, and 0.1M citric acid was added to adjust pH to about 7 to obtain a brucine gel formulation.

Comparative example 4

| Formula | Dosage (g) |
|---------|------------|
| Brucine | 10.0 |
| Poloxamer | 300.0 |
| absolute ethanol | 200.0 |
| Propylene glycol | 300.0 |
| 0.1M citric acid | 0.06 |
| Water | 1190.0 |

Preparation method:

(1) 300 g of poloxamer was added into water to swell completely to obtain gel matrix I; (2) 10 g of brucine was dissolved in absolute ethanol and propylene glycol, mixed with water at a balanced amount in the formula and stirred evenly to obtain brucine solution II; (3) the gel matrix I was mixed with the brucine solution II and stirred evenly, and 0.1M citric acid was added to adjust pH to about 7 to obtain a brucine gel formulation.

Test Example 1: Study on the appearance of the brucine gel formulation of the present disclosure
The physical appearance of the gel formulations prepared according to Examples 1-10 and Comparative Examples 1-4 were observed by visual inspection.

Table 1: Physical appearances of gel formulations

| Gel | Visual inspection |
|-----|-------------------|
| Example 1 | Moderate viscosity |
| Example 2 | Moderate viscosity |
| Example 3 | Moderate viscosity |
| Example 4 | Moderate viscosity |
| Example 5 | Moderate viscosity |
| Example 6 | Moderate viscosity |
| Example 7 | Moderate viscosity |
| Example 8 | Moderate viscosity |
| Example 9 | Moderate viscosity |
| Example 10 | Moderate viscosity |
| Comparative Example 1 | Moderate viscosity |

(continued)

| Gel | Visual inspection |
|---|---|
| Comparative Example 2 | Moderate viscosity |
| Comparative Example 3 | Moderate viscosity |
| Comparative Example 4 | Moderate viscosity |

The results show that all products met the viscosity requirements of gel formulations.

Test Example 2: Study on the pharmacokinetics of the brucine gel formulation of the present disclosure skin-administered to the joint fluid

1. Laboratory animals, drugs and instruments

1.1 Laboratory animals

Wistar rats, SPF grade (specific pathogen free laboratory animals): weight 210-250 g, female, animal age 10-11 weeks, purchased from SiPeiFu (Beijing) Biotechnology Co., Ltd.; license number: 11401500036047.

Animal room: During the experiment, the animals were kept in Beijing IncreasePharm Safety and Effectiveness Research Co., Ltd., experimental facility license: SYXK (JING) 2017-0026; facility management followed the national standard of the People's Republic of China GB14925-2001 "Experimental Animal Environment and Facilities".

Feeding conditions: artificial light for a 12-hour light-dark cycle, an ambient temperature maintained at 20-24°C, a humidity of 40%-70%, and air exchange 15 times per hour; the animals were kept in polycarbonate mouse cages, and 6 rats of the same sex in the same group were kept in each cage; the animal cage and litter were cleaned every 2 days.

Feed: Growth maintenance feed for rats and mice, purchased from SiBeiFu (Beijing) Biotechnology Co., Ltd.

Drinking water: drinking water for laboratory animals, which can be taken freely by animals, and new water bottles and fresh water were replaced every day.

1.2 Drugs

According to the preparation methods of Example 3, Example 4, Example 5, and Comparative Examples 1-4, Gel 1, Gel 2, Gel 3, Gel 4, Gel 5, Gel 6, and Gel 7 were obtained, respectively.

1.3 Instruments and reagents

Instruments:

Triple Quadrupole-Ion Trap Mass Spectrometer, AB Sciex QTRAP5500 Applied Biosystem; Liquid Chromatography: Ultra High Performance Liquid Chromatography, ExionLC AC Applied Biosystem Company; Microdialysis Sampling Equipment: CMA Microdialysis Sampling System

Reagents and drugs:

chloral hydrate, manufacturer: Shanghai Shanpu Chemical Co., Ltd.

1.4 Analytical method

1.4.1 Analytical method of LC-MS/MS to determine the drug concentration of brucine in the joint fluid of rats

The concentration of brucine in the dialysate samples was analyzed by Beijing IncreasePharm Safety and Effectiveness Research Co., Ltd., and the analysis used the non-confirmed liquid chromatography-tandem mass spectrometry method (LC-MS/MS). The standard curve range of Gels 1-7 was 0.2 to 200 ng/mL, and the lower limit of quantification was 0.2 ng/mL.

After adding internal standard warfarin to rat joint cavity dialysate, LC-MS/MS analysis was performed.

10 μL of the dialysate sample to be tested was directly taken and added with 2 μL of acetonitrile for processing. The standard curve (at a concentration of 0.20 ng/mL, 0.60 ng/mL, 2.00 ng/mL, 6.00 ng/mL, 20.0 ng/mL, 60.0 ng/mL, and 200 ng/mL respectively) (except for Double Blank), quality control and samples to be tested were added with 100 μL of internal standard (warfarin, 100 ng/mL), and vortexed to mix well; 10 μL of Double Blank sample was taken and added with 102 μL of acetonitrile, and vortexed to mix well; the supernatant was transferred, placed in the sample tray in order, and injected for determination. All dialysates were stored in a refrigerator at -80°C.

HPLC conditions:

HPLC column: ACQUITY UPLC BEH C8 1.7um 2.1×50mm Colume (Waters)

Flow rate: 0.5 mL/min; mobile phase A: water/formic acid (99.9/0.1, v/v)

Mobile phase B: Acetonitrile/formic acid (99.9/0.1, v/v); Injection volume: 10 µL; Column temperature: 40°C

Running time: 3.0 minutes

MS conditions:

Ion source: ESI; scan type: positive ion MRM (multiple reaction monitoring);

CUR Gas: 30.00; Spray voltage: 5500.00; Source temperature: 500°C;

GS1: 55.00; GS2: 55.00; CAD: Medium

Table 2: Monitoring ion parameters of brucine and internal standard

| - | Q1 mass (amu) | Q3 mass (amu) | Retention time (msec) | DP | CE | CXP |
|---|---|---|---|---|---|---|
| brucine | 395.2 | 244.1 | 100 | 110 | 51 | 12 |
| warfarin | 309.1 | 163.1 | 100 | 100 | 19 | 12 |

Calculation:
A linear equation (standard curve) was fitted between the peak area ratio of brucine and the internal standard and concentration at a weight of $1/\times2$ fitted by Linear program of Analyst 1.6.3 software. This equation was used to calculate the concentration of brucine in the dialysate.

1.4.2 Analysis of pharmacokinetic data

The plasma concentration-time data of individual animals were analyzed with WinNonlin software (professional edition, version 5.2; Pharsight). The non-compartmental model was used for concentration analysis. The pharmacokinetic parameters in the present disclosure are summarized as follows:

Cmax (ng/mL): Maximum (peak) dialysate drug concentration

Tmax (h): Peak time

t1/2(h): Half-life

AUC0-inf (ng*h/mL): Area under the drug concentration-time curve after administration of the test sample

2. Experimental method

Male SD rats were randomly divided into 4 groups with 12 rats in each group.

The study was divided into three groups, each group of animals was given a single transdermal administration (Gels 1-7) with a dose of 10 mg/kg. Animals do not need to fast before the experiment.
After the administration of the animals in each group, the microdialysis sampling device was adjusted to sample at a flow rate of 1 µL/min. Before sample collection, the probe was perfused and equilibrated for 1 h, and one sample was collected every 30 minutes and the sampling was performed for 10 h (during sampling, the rats were placed on an electric blanket at 38°C to observe the breathing rate, and the temperature was adjusted). The dialysate was stored at -80°C until analysis. During the experiment, the animals were anesthetized with chloral hydrate. If the rat was awake during the collection process, 0.3 mL of 10% chloral hydrate (intraperitoneal injection) was supplemented.

Table 3: Summary of the main pharmacokinetic parameters of the brucine gel skin-administered to the joint fluid

| Gel group | Gel 1 | Gel 2 | Gel 3 | Gel 4 | Gel 5 | Gel 6 | Gel 7 |
|---|---|---|---|---|---|---|---|
| Dosage (mg/kg) | 10 | 10 | 10 | 10 | 10 | 10 | 10 |
| Elimination half-life $t_{1/2}$ (h) | 2.12 | 1.54 | 3.39 | 1.56 | 1.67 | 1.66 | 1.73 |
| Peak time Tmax (h) | 1.25 | 1.75 | 4.50 | 1.37 | 1.75 | 2.13 | 1.68 |
| Peak concentration Cmax (ng/mL) | 2834 | 655 | 1553 | 435 | 495 | 417 | 472 |
| Area under the drug concentration-time curve $AUC_{0-inf}$ (ng*h/mL) | 1656 | 484 | 932 | 342 | 366 | 282 | 353 |

Gel 1, Gel 2, and Gel 3 all had good pharmacokinetic properties, and the exposure amount (AUC) of Gel 1 was higher than that of Gel 2 and Gel 3. It can be seen that Gel 1 had better pharmacokinetic properties with fast absorption, high exposure, and good transdermal effect; unexpectedly, we found strychnine-containing brucine (that is, total alkaloids of *Strychnos nux-vomica* L.) (Gel 5, Comparative Example 2) had a transdermal effect significantly lower than that of the gel prepared from single brucine (Gel 1, Example 3).

Test Example 3: treatment effect of brucine gel formulation of the present disclosure on joint swelling

1. Experimental animals and drugs

1.1 Experimental animals and drugs were the same as Test Example 2

1.2 Drugs

Complete Freund's Adjuvant (CFA), manufacturer: Beyotime Biotechnology Co., Ltd.
Voltaren (diclofenac diethylamine latex), manufacturer: Beijing Novartis Pharmaceutical Co., Ltd.
Gel 1, Gel 2, Gel 3, and Gel 5 obtained according to the preparation methods of Example 3, Example 4, Example 5, and Comparative Example 2 respectively.

2. Experimental method

After the animals were anesthetized with an anesthesia machine, 0.1 ml of complete Freund's adjuvant (CFA) was injected intracutaneously into the left hind toe of each rat, in which the concentration of Bacille Calmette-Guerin (BCG) was 10 mg/ml, to induce the occurrence of arthritis.
According to the clinical score of rat arthritis, they were randomly divided into blank control group, model control group, positive control group (Voltarin, 50 mg/kg), Gel 1 group (5 mg/kg), Gel 2 group (5 mg/kg), Gel 3 group (5 mg/kg), and Gel 5 group (5 mg/kg).
Different amounts of gel were weighed and applied around the left foot and the ankle joint of the rat, which was gently massaged for a few times, wrapped with release paper, sticked with a layer of medical plaster, and then wrapped with medical plaster to prevent the drug from falling off. After 4 h, the medical plasters were removed, and the left foot of the rat was washed. All groups were administered once a day for 7 consecutive days, and the start time of the administration was basically the same every day.
Arthritis clinical score: once before modeling, once during grouping, and once after grouping. Using the 0-4 grade scoring method, the right hind foot and left hind foot were measured, and the highest score for each foot was 4 points. The scoring standards are as follows:

0 point: no redness and swelling

1 point: red and swollen toe joints and mild red and swollen feet

2 points: swelling of toe joints and toes

3 points: swelling of the foot below the ankle joint

4 points: swelling of the entire foot including the ankle joint

Swelling rate determination: The thickness of the left hind foot plantar was measured once before modeling, once before grouping, and once a week after administration to calculate the swelling rate.

$$\text{Swelling rate} = (\text{measured data each time after modeling} - \text{the data before modeling}) / \text{the data before modeling} \times 100\%$$

The results are as follows:

Table 4: Results of the swelling rate of joint swelling treated by brucine gel formulation

| No. | Group | Dose | Toe swelling rate | Toe joint swelling rate | |
| --- | --- | --- | --- | --- | --- |
| | | | | Right hind foot | Left hind foot |
| G1 | Blank control group | - | 4.71±0.18 | 0.00±0.00 | 0.00±0.00 |
| G2 | Model control group | - | 7.46±0.83♦♦ | 1.75±1.41♦♦ | 3.50±1.00♦♦ |
| G3 | Positive control group | 50 mg/kg | 5.69±0.13■■ | 0.50±0.5" | 2.75±0.50■ |
| G4 | Gel 1 group (Example 3) | 5.0 mg/kg | 5.77±0.38■■ | 0.50±0.16■■ | 2.25±0.50■ |
| G5 | Gel 2 group (Example 4) | 5.0 mg/kg | 7.03±0.75 | 0.75±0.58■■ | 3.00±1.16■ |
| G6 | Gel 3 group (Example 5) | 5.0 mg/kg | 6.36±0.38■ | 1.50±1.00 | 3.75±0.96 |
| G7 | Gel 5 group (Comparative Example 3) | 5.0 mg/kg | 6.73±0.69■ | 1.25±1.50■ | 3.00±1.41■ |
| Compared with the blank group: ♦♦$P<0.01$, ♦$P<0.05$; compared with the model group: ■■$P<0.01$, ■$P<0.05$ | | | | | |

It can be seen from the table that after intradermal injection of 0.1 ml complete Freund's adjuvant on the left plantar of the rat, the toe swelling rate and left hind toe joint swelling rate of the model group (G2) were significantly different from those of the blank group ($P<0.01$), indicating that the model was successfully established;

Transdermal administration of Voltaren (Diclofenac sodium diethylamine; positive control group G3) and brucine gel (Example 3, Gel 1 group; Example 4, Gel 2 group; Example 5, Gel 3 group), gel of total alkaloids of *Strychnos nux-vomica* L. (Comparative Example 2, Gel 5 group) can all reduce the swelling, where Gel 1 group was significantly better than the other groups.

**Claims**

1. A brucine gel formulation, wherein by weight percentage, the gel formulation comprises 0.5%-1% of brucine, 0.5%-3% of gel matrix material and 10%-30% of a co-solvent.

2. The gel formulation according to claim 1, wherein the gel matrix material is one of carbomer, sodium carboxymethyl cellulose, hydroxypropyl methyl cellulose, sodium alginate, gum arabic, gum tragacanth, gelatin, chitosan, polyacrylic acid and sodium salt thereof, or a combination thereof.

3. The gel formulation according to claim 1 or 2, wherein the gel matrix material is selected from the group consisting of carbomer, sodium carboxymethyl cellulose, hydroxypropyl methyl cellulose, gum arabic and sodium polyacrylate.

4. The gel formulation according to any one of claims 1 to 3, wherein the gel matrix material is selected from sodium polyacrylate, carbomer and hydroxypropyl methylcellulose.

5. The gel formulation according to any one of claims 1 to 4, wherein the gel matrix material is hydroxypropyl methylcellulose.

6. The gel formulation according to any one of claims 1-5, wherein the co-solvent is one of ethanol, benzyl alcohol, propylene glycol, glycerin, polyethylene glycol 400 and isopropanol, or a combination thereof.

7. The gel formulation according to any one of claims 1-6, wherein the gel formulation further comprises a pH adjusting agent, and the pH adjusting agent is selected from the group consisting of an organic amine, sodium hydroxide, sodium bicarbonate, sodium carbonate, acetic acid, citric acid and phosphate buffer; preferably, the organic amine is selected from triethanolamine, triethylamine, diethylamine and laurylamine.

8. The gel formulation according to any one of claims 1-7, wherein, by weight percentage, the gel formulation further comprises 0.01%-0.1% of an antioxidant, and the antioxidant is one of anhydrous sodium sulfite, sodium metabisulfite, sodium bisulfite, sodium thiosulfate, tert-butyl *p*-hydroxyanisole, dibutyl phenol, vitamin C, vitamin C palmitate, and ethylenediaminetetraacetic acid and sodium salt thereof, or a combination thereof.

9. The gel formulation according to any one of claims 1-8, wherein, by weight percentage, the gel formulation further comprises 0.01%-0.15% of a bacteriostatic agent, and the bacteriostatic agent is one of chlorobutanol, ethyl paraben, benzyl alcohol, methyl paraben, butyl paraben, sorbic acid and potassium salt thereof, and benzoic acid and sodium salt thereof, or a combination thereof.

10. The gel formulation according to any one of claims 1-9, wherein by weight percentage, the gel formulation comprises 68%-80% of water.

11. A preparation for preparing the gel formulation of any one according to claims 1-10, comprising steps of: (1) swelling the gel matrix material to obtain a gel matrix I; (2) dissolving brucine and a co-solvent in water to obtain a brucine solution II; (3) mixing the gel matrix I and the brucine solution II to obtain the brucine gel formulation.

12. The preparation method according to claim 11,

    wherein, step (1) can further comprise adding a pH adjusting agent to adjust pH value to 5.5-6.5;
    step (2) can further comprise adding an antioxidant, a bacteriostatic agent and a transdermal agent; and
    step (3) can further comprise adding a pH adjusting agent to adjust pH value to 6.5-7.5.

13. Use of the brucine gel formulation according to any one of claims 1-10 in the manufacture of a medicament for treating knee osteoarthritis.

## INTERNATIONAL SEARCH REPORT

| International application No. |
|---|
| **PCT/CN2020/106108** |

| | |
|---|---|
| **A.** | **CLASSIFICATION OF SUBJECT MATTER** |

A61K 9/06(2006.01)i;  A61K 47/38(2006.01)i;  A61K 47/36(2006.01)i;  A61K 47/42(2017.01)i;  A61K 47/32(2006.01)i;  A61K 31/475(2006.01)i;  A61P 19/02(2006.01)i;  A61P 29/00(2006.01)i

According to International Patent Classification (IPC) or to both national classification and IPC

| | |
|---|---|
| **B.** | **FIELDS SEARCHED** |

Minimum documentation searched (classification system followed by classification symbols)

A61K9/-;A61K47/-;A61P19/-;A61P29/-

Documentation searched other than minimum documentation to the extent that such documents are included in the fields searched

Electronic data base consulted during the international search (name of data base and, where practicable, search terms used)

CNTXT, CNABS, CNKI, DWPI, VEN, STNext, WEB OF SCIENCE, 读秀学术搜索: 北京盈科瑞创新医药股份有限公司, 北京因科瑞, 马钱子碱, 马钱子生物碱, 凝胶, 骨架材料, 卡波姆, 羟丙基甲基纤维素, 聚丙烯酸钠, 骨关节炎, 关节炎, Brucine, hyderogel, gel, matrix

| | |
|---|---|
| **C.** | **DOCUMENTS CONSIDERED TO BE RELEVANT** |

| Category* | Citation of document, with indication, where appropriate, of the relevant passages | Relevant to claim No. |
|---|---|---|
| X | CN 102008476 A (NANJING UNIVERSITY OF CHINESE MEDICINE) 13 April 2011 (2011-04-13)<br>    claim 4 | 1-13 |
| X | 张卫华 等 (ZHANG, Weihua et al.). "不同促渗剂对马钱子碱囊泡凝胶体外渗透性能的影响 (Effects of Different Penetration Enhancers on in Vitro Permeability of Brucine Vesicles Gels)"<br>《中国实验方剂学杂志》 (Chinese Journal of Experimental Traditional Medical Formulae), Vol. 21, No. 7, 30 April 2015 (2015-04-30),<br>    pp. 14-16 | 1-13 |
| X | 陈志鹏 等 (CHEN, Zhipeng et al.). "用于关节腔注射的马钱子碱壳聚糖温敏凝胶的研究 (Brucine Chitosan Thermosensitive Hydrogel for Intra-Articular Injection)"<br>药学学报 (Acta Pharmaceutica Sinica), Vol. 47, No. 5, 31 December 2012 (2012-12-31),<br>    pp. 652-656 | 1-13 |

| ✓ Further documents are listed in the continuation of Box C. | ✓ See patent family annex. |
|---|---|

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **16 October 2020** | **04 November 2020** |

| Name and mailing address of the ISA/CN | Authorized officer |
|---|---|
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

## INTERNATIONAL SEARCH REPORT

| International application No. |
| --- |
| **PCT/CN2020/106108** |

### C. DOCUMENTS CONSIDERED TO BE RELEVANT

| Category* | Citation of document, with indication, where appropriate, of the relevant passages | Relevant to claim No. |
| --- | --- | --- |
| X | 邓向涛 等 (DENG, Xiangtao et al.). "马钱子碱固体脂质纳米粒凝胶骨架缓释片的研制 (Study on Matrix Sustained Release Tablets of Brucine Solid Lipid Nanoparticles)" 中草药 (Chinese Traditional and Herbal Drugs), Vol. 49, No. 22, 30 November 2018 (2018-11-30), pp. 5298-5304 | 1-12 |
| X | WU Zhenzhen et al.,. "Tissue distribution and pharmacokinetics of brucine niosomal gels in rats after topical and oral application" 《Journal of Chinese Pharmaceutical Sciences》, Vol. 27, No. 2,, 31 December 2018 (2018-12-31), pages 92-98 | 1-13 |
| X | WU, P. et al.,. "A Novel Brucine Gel Transdermal Delivery System Designed for Anti-Inflammatory and Analgesic Activities," 《INTERNATIONAL JOURNAL OF MOLECULAR SCIENCES》, Vol. 18, No. 4, 03 April 2017 (2017-04-03), Document Number 757 | 1-13 |
| Y | 张卫华 等 (ZHANG, Weihua et al.). "不同促渗剂对马钱子碱囊泡凝胶体外渗透性能的影响 (Effects of Different Penetration Enhancers on in Vitro Permeability of Brucine Vesicles Gels)" 《中国实验方剂学杂志》 ( 《Chinese Journal of Experimental Traditional Medical Formulae》 ), Vol. 21, No. 7, 30 April 2015 (2015-04-30), pp. 14-16 | 13 |
| Y | 邓向涛 等 (DENG, Xiangtao et al.). "马钱子碱固体脂质纳米粒凝胶骨架缓释片的研制 (Study on Matrix Sustained Release Tablets of Brucine Solid Lipid Nanoparticles)" 中草药 (Chinese Traditional and Herbal Drugs), Vol. 49, No. 22, 30 November 2018 (2018-11-30), pp. 5298-5304 | 13 |

Form PCT/ISA/210 (second sheet) (January 2015)

INTERNATIONAL SEARCH REPORT
Information on patent family members

International application No.

**PCT/CN2020/106108**

| Patent document cited in search report | | | Publication date (day/month/year) | Patent family member(s) | | | Publication date (day/month/year) |
|---|---|---|---|---|---|---|---|
| CN | 102008476 | A | 13 April 2011 | CN | 102008476 | B | 07 January 2015 |

Form PCT/ISA/210 (patent family annex) (January 2015)

**REFERENCES CITED IN THE DESCRIPTION**

**Patent documents cited in the description**

- CN 201910709653X

- GB 149252001 A